# EUROPÄISCHE PATENTSCHRIFT

(11) **EP 0 535 315 B1**
(45) Veröffentlichungstag und Bekanntmachung des Hinweises auf die Patenterteilung: **15.06.1994**
(21) Anmeldenummer: 92111117.5
(22) Anmeldetag: 01.07.1992
(51) Int. Cl.: A61B 17/60, A61F 2/44

(54) **Wirbelkörperplatzhalter**
Vertebral spacer
Dispositif pour maintenir l'écartement entre vertèbres

(30) Priorität: 30.09.1991 DE 9112176 U
(43) Veröffentlichungstag der Anmeldung: 07.04.1993
(73) Patentinhaber: Howmedica GmbH, D-24232 Schönkirchen (DE)
(72) Erfinder: Metz-Stavenhagen, Peter, Dr. Med., W-3590 Bad Wildungen (DE)
(74) Vertreter: Patentanwälte Hauck, Graalfs, Wehnert, Döring, Siemons

(56) Entgegenhaltungen:
- EP-A- 0 188 954
- EP-A- 0 328 883
- DE-U- 9 006 646
- DE-U- 9 107 494
- FR-A- 2 651 992

## Beschreibung

Gegenstand der Erfindung ist ein Wirbelkörperplatzhalter nach dem Oberbegriff des Anspruches 1. Ein derartiger Wirbelkörperplatzhalter ist aus EP-A-0 188 954 bekannt.

Ein derartiger Platzhalter soll zwei Wirbel nach Entfernen mindestens eines zwischenliegenden Wirbels, beispielsweise wegen Tumorentwicklung, in einem konstanten Abstand voneinander fixieren. Zur weiteren Überbrückung des Abstands zwischen den Wirbeln können zusätzlich autologe Knochenspäne oder alloplastischer Ersatz eingebracht werden.

Aus der EP-A-0 328 883 ist schon eine Stützvorrichtung für die menschliche Wirbelsäule bekannt, bei der beidseitig in eine Gewindehülse Gewindebolzen eingeschraubt sind. Die Bolzenköpfe weisen Klemmflächen auf, die mit Klemmflachen von Pedikelschrauben und Schraubverbindungen zur Lagefixierung der Pedikelschrauben an den Gewindebolzen zusammenwirken. Bei dieser Stützvorrichtung bilden Gewindehülsen und Gewindebolzen eine Einstellvorrichtung, mit der der Abstand der Pedikelschrauben und somit der diese in den Pedikel aufnehmenden Wirbelkörper einstellbar ist. Die Einstellvorrichtung liegt außerhalb der Wirbelsäule, so daß sie bei Überbrückung fehlender Wirbel einer relativ ungünstigen statischen und dynamischen Belastung unterworfen ist. Damit die Konstruktion die erforderliche Sicherheit aufweist, ist sie deshalb in der Ausführung relativ kostenaufwendig.

Ein Implantat zum Einsetzen zwischen Wirbelkörpern der Wirbelsäule der eingangs genannten Art ist aus der DE-C3-30 23 942 bekannt. Demnach weist eine Einstellvorrichtung aus Gewindehülse und Gewindebolzen an den Enden von Gewindehülse und Gewindebolzen Stützkörper in Form von Tellern und Vorsprüngen auf, die auf die Innenflächen in ihrer gegenseitigen Lage zu fixierender Wirbelkörper einwirken. Diese Konstruktion wird zwar günstiger belastet als die Pedikelschrauben-Vorrichtungen, ist jedoch nur entlang ihrer Gewindelängsachse verstellbar. Dies ist auch nur relativ begrenzt möglich, weil Gewindebolzen und Gewindehülse aufgrund ihrer ovalen Querschnittsgestalt nur abschnittsweise ineinandergreifen.Ein Kippen der abgestützen Wirbel relativ zueinander, wie es in bestimmten Bereichen der Wirbelsäule auch aufgrund krankhafter Verförmungen erwünscht sein kann, ist bei diesem Platzhalter nicht möglich.

Dasselbe gilt für das Implantat gemäß DE-C2-37 29 600, bei dem die Stützkörper Winkelprofile haben, um zweck Verschraubung an den Seiten der abzustützenden Wirbelkörper zur Anlage zu gelangen. Für eine Justierung des Stützkörpers sind hier die Durchbrechungen für die Knochenschraube langlochförmig ausgebildet. Ein Nachjustieren dürfte jedoch nur in sehr engen Grenzen möglich und in der Durchführung problematisch sein, zumal zusätzliche Dorne für die Stützkörperfestlegung vorgesehen sind.

Schließlich ist aus der DE-U1-91 01 603.7 ein Wirbelkörperimplantat mit durch Schraubbolzen relativ zueinander verstellbaren Stützflächen bekannt, dessen Länge in Schraubrichtung mittels einsteckbarer Platzhalter variabel ist. Bei diesem in Konstruktion und Bevorratung aufwendigen Implantat ist für das Verstellen der Stützflächen ebenfalls nur ein Freiheitsgrad gegeben.

Davon ausgehend liegt der Erfindung die Aufgabe zugrunde, einen die Justierbarkeit gegeneinander abzustützender Wirbelkörper verbessernden Wirbelkörperplatzhalter zu schaffen, der günstiger bevorratet werden kann.

Die Lösung dieser Aufgabe ist im Anspruch 1 angegeben. Vorteilhafte Ausgestaltungen sind den Unteransprüchen entnehmbar.

Bei einem erfindungsgemäßen Platzhalter ist durch die Verbindung der Einstellvorrichtung mit den Stützkörpern über Klemmflächen eine weitere Einstellmöglichkeit gegeben. Die Stützflächen können nämlich relativ zur Achse von Hülse und Bolzen gekippt werden, wobei die relative Drehbarkeit von Bolzen und Hülse eine Kippbewegung in beliebigen Raumrichtungen ermöglicht. Die damit verbundene, relative Axialverschiebung der Stützflächen infolge des Gewindeeingriffs zwischen Bolzen und Hülse kann wegen ihrer Geringfügigkeit hingenommen werden. Überdies ermöglicht der erfindungsgemäße Platzhalter den Rückgriff auf Bauteile von Pedikelschrauben-Vorrichtungen gemäß EP-A-0 328 883, wie Hülse und Bolzen mit Klemmfläche sowie zugehörige Arretierungsvorrichtungen, weil dieser Technik zumindest eine Einsatzdomäne bei der Wirbelsäulenkorrektur verbleibt.

Erfindungsgemäß kommen prinzipiell nur die Stützkörper mit den Klemmflächen hinzu, so daß gegenüber herkömmlichen Wirbelkörperplatzhaltern der Vorteil der Modularität mit dem Pedikelschrauben-System gegeben ist. Dem geringen Wirbelabstand bei Ersatz nur eines Wirbelkörpers wird man durch kurze Hülsen bzw. Bolzen Rechnung tragen.

Bei einer Hülse mit zwei Bolzen ist die Variabilität der Stützkörper-Einstellung in Axial- und Kipprichtung verbessert. Den Reib- bzw. Formschluß der Klemmflächen begünstigende Maßnahmen tragen zur Sicherheit der Wirbelkörperabstützung bei. Werkzeugangriffsflächen der Hülse ermöglichen eine schnelle Anpassung der Einstellvorrichtung an die individuellen Gegebenheiten des Patienten. Zwischen Bolzen und Hülse wirkende Feststellmittel, insbesondere mit einer Scheibe, tragen zur Sicherung der Abstützung in Axialrichtung bei.

Stützplatten als Stützkörper verringern die axiale Baulänge und begünstigen den Ersatz nur eines Wirbelkörpers. In Anpassung an die Form der abzustützenden Wirbelkörper kann den Stützflächen eine runde oder ovale Form gegeben sein. Aus den Stützflächen herausragende Zapfen bewirken eine Verankerung in den Wirbelkörpern, welche die Platzhalter in der belasteten Wirbelsäule festhält. Das Eindringen in das relativ weiche Wirbelmaterial wird durch abgerundete Zapfenenden begünstigt.

Weitere Einzelheiten und Vorteile des Gegenstandes der Erfindung ergeben sich aus der nachfolgenden Beschreibung der zugehörigen Zeichnungen, die bevorzugte Ausführungsformen zeigen. In den Zeichnungen zeigen:
- Fig. 1: Hülse mit Bolzen des Wirbelkörperplatzhalters im Teilschnitt;
- Fig. 2: zugehöriger Stützkörper desselben Wirbelkörperplatzhalters in etwas vergrößerter Seitenansicht;
- Fig. 3: derselbe Stützkörper entlang der Linie III-III der Fig. 2 geschnitten;
- Fig. 4: derselbe Stützkörper in einer Ansicht auf die der Einstellvorrichtung zugewandten Seite;
- Fig. 5: derselbe Stützkörper in einer Ansicht auf die dem Wirbel zugewandte Seite;
- Fig. 6: ein anderer Stützkörper für kleinere Wirbel in einer Ansicht auf die der Einstellvorrichtung zugewandte Seite.

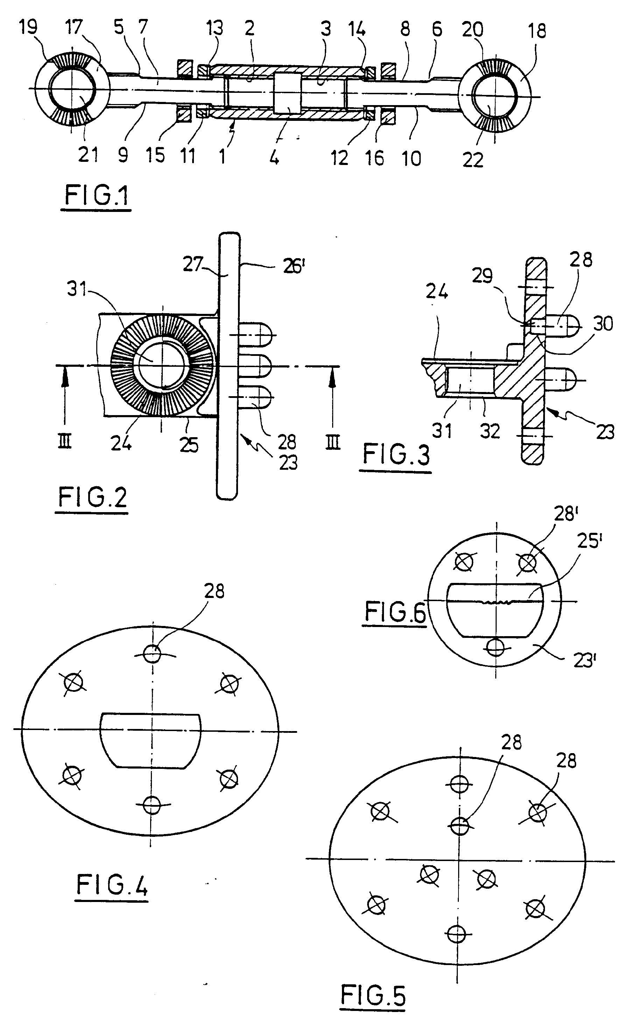

Gemäß Fig. 1 hat ein erfindungsgemäßer Wirbelkörperplatzhalter eine Hülse 1 mit zueinander gegenläufigen Innengewinden 2, 3, die durch einen im Durchmesser erweiterten Entlastungbohrungsabschnitt 4 voneinander getrennt sind.

In beide Enden der Gewindehülse 1 sind Bolzen 5, 6 mit Außengewinde 7, 8 eingeschraubt, die mit Abflachungen 9, 10 versehen sind. An den Abflachungen 9, 10 sind Scheiben 11, 12 geführt, die eine mit den Stirnseiten der Hülse 1 zusammenwirkende Verzahnung 13, 14 haben. Kontermuttern 15, 16 wirken mit den Außengewinden 7, 8 der Bolzen zusamen, um die Scheiben 11, 12 über die Verzahnungen 13, 14 an der Hülse 1 festzulegen und somit die Bolzen 5, 6 zu sichern.

An ihren Enden haben die Bolzen 5, 7 Klemmflächen 17, 18, die parallel zu den Bolzenachsen ausgerichtet sind und zumindest segmentweise eine Zahnung 19, 20 haben. Diese Klemmflächen 17, 18 bildenden Bolzenköpfe haben ferner Gewindebohrungen 21, 22, die der Aufnahme von - nicht dargestellten - Schraubenbolzen zur Festlegung nachfolgend zu erläuternder Stützkörper aufweisen.

Gemäß Fig. 2 sind Stützkörper 23 mit einer den Zahnungen 19, 20 entsprechenden Zahnung 24 an einer Klemmfläche 25 versehen, die sich senkrecht zur Stützfläche 26 einer Stützplatte 27 erstreckt. Über die Stützfläche 26 stehen Zapfen 28 mit abgerundeten Enden hervor, die gemäß Fig. 3 mit einem Befestigungszapfen 29 in Bohrungen 30 der Stützplatte 27 verankert, z.B. verpreßt sind.

Der Stützkörper 23 hat gemäß Fig. 2 und 3 eine Bohrung 31 zur Aufnahme eines Schraubenbolzens zwecks Verbindung mit den Gewindebohrungen 21, 22 der Bolzen 5, 6. Der Kopf des - nicht dargestellten - Schraubenbolzens ist konisch und wirkt mit einem konischen Bohrungsabschnitt 32 der doppelkonischen Bohrung 31 zusammen.

Aus den Fig. 4 und 5 ist ersichtlich, daß der Stützkörper in Anpassung an die Wirbel des unteren Bereichs der Wirbelsäule eine ovale Kontur hat und in Randnähe sechs gleichmäßig verteilte Bohrungen 28 zur temporären Befestigung aufweist. Ein zweiter Ring von drei gleichmäßig verteilten Zapfen 28 ist in Zentrumsnähe angeordnet.

Gemäß Fig. 6 kann ein Stützkörper 23′ für kleinere Wirbel mit dem oberen Wirbelsäulenbereich eine runde Kontur mit nur drei gleichmäßig verteilten Zapfen 28′ in Randnähe aufweisen. Die Ausbildung der Klemmfläche 25′ stimmt mit derjenigen des Stützkörpers 23 überein, weil Modularität mit den Klemmflächen 17, 18 der Bolzen 5, 6 gemäß Fig. 1 gegeben sein soll.

Ein erfindungsgemäßer Wirbelkörperplatzhalter wird somit durch Auswahl von Stützkörpern 23 und deren Anbringung an den mit der Hülse 1 vormontierten Bolzen 5, 6 mittels Schraubenbolzen fertiggestellt. Nach dem Einsatz zwischen zwei abzustützende Wirbel vor Ausrichtung der Stützplatten 27 werden diese durch Verdrehen der Hülse 1 auseinandergeschoben bis die Wirbel den gewünschten Abstand in Längsrichtung erhalten.Eine genaue Ausrichtung der Wirbelkörper kann durch Kippen der Stützplatten 27 und ggf. geringfügiges Drehen der Bolzen 5, 6 erfolgen. Schließlich werden die Klemmflächen 17, 18, 25, 25′ durch Anziehen der Schraubenbolzen sowie die Bolzen 5, 6 durch Anziehen der Kontermuttern 15, 16 gesichert, wodurch eine stabile und sichere Abstützung der Wirbelkörper erzielt wird.

Zusätzlich zum Wirbelkörperplatzhalter kann eine Fixierung mit einem Fixationssystem an der Wirbelsäule erfolgen.

## Patentansprüche

1. Wirbelkörperplatzhalter mit einem ersten und einem zweiten Stützkörper (23, 23′), die über eine Stützfläche (26) gegen die Lagerflächen von benachbarten Wirbelkörpern anlegbar sind und einer Einstellvorrichtung mit einer Hülse (1) mit Innengewinde (2, 3) sowie damit zusammenwirkenden Bolzen (5, 6) mit Außengewinde zum Einstellen der Stützkörper (23, 23′) in einem bestimmten Abstand voneinander, dadurch gekennzeichnet, daß die plattenförmigen Stützkörper (23, 23′) auf der dem zugeordneten Wirbelkörper abgewandten Seite eine koaxiale oder achsparallele Klemmfläche (25, 25′) aufweisen und die Bolzen (5, 6) ihrerseits axiale oder achsparallele Klemmflächen (17, 18) haben, die mit den Klemmflächen der Stützkörper zusammenwirken und eine Schraubverbindung vorgesehen ist, die die Klemmflächen von Einstellvorrichtung und Stützkörper (23, 23′) in ihrer Lage klemmend gegeneinander festlegt.

2. Platzhalter nach Anspruch 1, dadurch gekennzeichnet, daß die Klemmflächen eine große Rauhigkeit aufweisen.

3. Platzhalter nach Anspruch 1, dadurch gekennzeichnet, daß die Klemmflächen (17, 18, 25, 25') eine Zahnung (19, 20, 24) aufweisen.

4. Platzhalter nach einem der Ansprüche 1 bis 3, dadurch gekennzeichnet, daß die Klemmflächen (17, 18, 25) kreisförmig sind.

5. Platzhalter nach einem der Ansprüche 1 bis 4, dadurch gekennzeichnet, daß die Klemmflächen (25, 25′) der Stützkörper (23, 23′) von Bohrungen (31) durchsetzt sind und die Schraubverbindung einen die Bohrungen durchsetzenden Schraubenbolzen aufweist.

6. Platzhalter nach Anspruch 5, dadurch gekennzeichnet, daß der Kopf des Schraubenbolzens konisch ist und mit einem konischen Bohrungsabschnitt (32) der Stützkörper (23) bzw. der Gewindebolzen zusammenwirkt.

7. Platzhalter nach Anspruch 6, dadurch gekennzeichnet, daß die Bohrung (31) der Stützkörper (23) doppelkonisch ist.

8. Platzhalter nach Anspruch 6 oder 7, dadurch gekennzeichnet, daß der Schraubenbolzenkopf versenkte Schlüsselflächen aufweist.

9. Platzhalter nach einem der Ansprüche 1 bis 8, dadurch gekennzeichnet, daß die Hülse (1) ebene Werkzeugangriffsflächen aufweist.

10. Platzhalter nach einem der Ansprüche 1 bis 9, gekennzeichnet durch mehrere mit den Bolzen (5, 6) und der Hülse (1) zusammenwirkende Feststellmittel (11, 12, 15, 16) zur Festlegung der axialen und Drehlage dieser Teile zueinander.

11. Platzhalter nach einem der Ansprüche 1 bis 10, dadurch gekennzeichnet, daß eine Kontermutter (15, 16) auf dem Gewinde (7, 8) der Bolzen (5, 6) sitzt.

12. Platzhalter nach Anspruch 10 und 11, dadurch gekennzeichnet, daß das Gewinde (7, 8) des Bolzens (5, 6) mindestens eine Abflachung (9, 10) aufweist, zwischen Kontermutter (15, 16) und Hülse (1) eine an der Abflachung unverdrehbar geführte Scheibe (11, 12) auf dem Bolzen sitzt, die eine stirnseitige, mit einer Stirnseite der Hülse (1) zusammenwirkende Verzahnung (13, 14) aufweist.

13. Platzhalter nach einem der Ansprüche 1 bis 12, dadurch gekennzeichnet, daß die Stützflächen (26) eine runde Kontur haben.

14. Platzhalter nach einem der Ansprüche 1 bis 12, dadurch gekennzeichnet, daß die Stützflächen (26) eine ovale Kontur haben.

15. Platzhalter nach einem der Ansprüche 1 bis 14, dadurch gekennzeichnet, daß die Stützkörper (23, 23′) über die Stützflächen hinausragende Zapfen (28, 28′) aufweisen.

16. Platzhalter nach Anspruch 15, dadurch gekennzeichnet, daß jeder Stützkörper (23, 23′) mehrere Zapfen in Rand-und/oder Zentrumsnähe der Stützfläche (26) hat.

17. Platzhalter nach einem der Ansprüche 15 oder 16, dadurch gekennzeichnet, daß jeder Zapfen (28, 28′) am freien Ende abgerundet ist.

## Claims

1. A vertebral spacer device comprising a first and a second supporting member (23, 23′) which can be brought into engagement with the mounting faces of adjacent vertebras through a supporting face, and an adjusting means including a sleeve (1) having an internal thread (2, 3) and a pair of bolts (5, 6) having an outer thread (7, 8) cooperating with said sleeve to adjust the supporting members (23, 23′) in a predetermined distance from each other, characterized in that the side of the plate-like supporting members (23, 23′) facing away from the associated vertebra comprise a clamping face (25, 25′) which is coaxial or disposed parallel to the axis, and that the bolts (5, 6) comprise clamping faces (17, 18) which are axial or disposed parallel to the axis, which clamping faces coact with the clamping faces of said supporting members and that a screwing connection is provided to fixedly press the clamping faces of said adjusting means and of said supporting members (23, 23′) together in a predetermined position.

2. The spacer device of claim 1, characterized in that the clamping faces are heavily roughened.

3. The spacer device of claim 1, characterized in that the clamping faces (17, 18, 25, 25′) include a toothing (19, 20, 24).

4. The spacer device of one of claims 1 to 3, characterized in that the clamping faces (17,18,25) are circular.

5. The spacer device of one of claims 1 to 4, characterized in that the clamping faces (25, 25′) of the supporting members (23, 23′) are provided with bores (31) and that the screwing connection includes a screw bolt extending through the bores.

6. The spacer device of claim 5, characterized in that the head of the screw bolt is conical cooperating with a conical bore section (32) of the supporting member (23) or, respectively the threaded bolt.

7. The spacer device of claim 6, characterized in that the bore (31) of the supporting member (23) is double-conical.

8. The spacer device of claim 6 or 7, characterized in that the screw bolt head is provided with a tool engaging recess.

9. The spacer device of one of claims 1 to 8, characterized in that the sleeve (1) is provided with flat tool engaging faces.

10. The spacer device of one of claims 1 to 9, characterized in that a plurality of fixing means (11, 12, 15, 16) cooperating with the bolt 85,6) and the sleeve (1) are provided to secure the set components together in axial and rotational position.

11. The spacer device of one of claims 1 to 10, characterized in that a counter nut (15, 16) is provided on the thread (7, 8) of the bolts (5, 6).

12. The spacer device of claims 10 and 11, characterized in that the thread (7, 8) of the bolts (5, 6) each include at least a flat face (9, 10) that a disc (11, 12) non-rotatably supported on the flat sides is placed on the bolt between the counter nut (15, 16) and the sleeve (1) which disc includes a front-sided toothing (13, 14) cooperating with a front face of the sleeve (1).

13. The spacer device of one of claims 1 to 12, characterized in that the supporting faces (26) are rounded.

14. The spacer device of one of claims 1 to 12, characterized in that the supporting faces (26) are oval shaped.

15. The spacer device of one of claims 1 to 14, characterized in that the supporting members (23, 23′) are provided with pins (28, 28′) extending beyond the supporting surfaces.

16. The spacer device of claim 15, characterized in that each supporting member (23, 23′) is provided with a plurality of pins near the edge and/or the center of the supporting face (26).

17. The spacer device of one of claims 15 or 16, characterized in that each pin (28, 28′) is rounded at its free end.

## Revendications

1. Dispositif de maintien de l'écartement entre des vertèbres, comprenant un premier et un second corps d'appui (23, 23′) qui peuvent être appliqués, par l'intermédiaire d'une surface d'appui (26), sur les surfaces de portée de vertèbres voisines, et un dispositif de réglage comportant un manchon (1) à filetage intérieur (2, 3), ainsi que, coopérant avec celui-ci, des broches (5, 6) à filetage extérieur, pour régler les corps d'appui (23, 23′) à un écartement déterminé l'un de l'autre,
caractérisé en ce que les corps d'appui (23, 23′), en forme de plaque, présentent sur le côté opposé à celui qui fait face à la vertèbre associée, une surface de serrage (25, 25′) coaxiale ou parallèle à l'axe, en ce que les broches (5, 6) comportent pour leur part des surfaces de serrage (17, 18) axiales ou parallèles à l'axe, qui coopèrent avec les surfaces de serrage des corps d'appui, et en ce qu'est prévue une liaison à vis qui fixe dans leur position, et de manière serrée les unes contre les autres, les surfaces de serrage du dispositif de réglage et des corps d'appui (23, 23′).

2. Dispositif de maintien de l'écartement selon la revendication 1, caractérisé en ce que les surfaces de serrage présentent une rugosité élevée.

3. Dispositif de maintien de l'écartement selon la revendication 1, caractérisé en ce que les surfaces de serrage (17, 18, 25, 25′) présentent une denture (19, 20, 24).

4. Dispositif de maintien de l'écartement selon l'une des revendications 1 à 3, caractérisé en ce que les surfaces de serrage (17, 18, 25) sont de forme circulaire.

5. Dispositif de maintien de l'écartement selon l'une des revendications 1 à 4, caractérisé en ce que les surfaces de serrage (25, 25′) des corps d'appui (23, 23′) sont traversées par des alésages (31), et en ce que la liaison à vis comprend un boulon fileté traversant les alésages.

6. Dispositif de maintien de l'écartement selon la revendication 5, caractérisé en ce que la tête du boulon fileté est conique et coopère avec un tronçon d'alésage conique (32) des corps d'appui (23) et ou des broches filetées.

7. Dispositif de maintien de l'écartement selon la revendication 6, caractérisé en ce que l'alésage (31) des corps d'appui (23) est doublement conique.

8. Dispositif de maintien de l'écartement selon la revendications 6 ou 7, caractérisé en ce que la tête du boulon fileté comporte des surfaces en dépression pour une clé.

9. Dispositif de maintien de l'écartement selon l'une des revendications 1 à 8, caractérisé en ce que le manchon (1) comporte des surfaces de prise planes pour un outil.

10. Dispositif de maintien de l'écartement selon l'une des revendications 1 à 9, caractérisé par plusieurs moyens de blocage (11, 12, 15, 16) coopérant avec les broches (5, 6), et destinés à fixer la position axiale et angulaire de ces pièces l'une par rapport à l'autre.

11. Dispositif de maintien de l'écartement selon l'une des revendications 1 à 10, caractérisé en ce qu'un contre-écrou (15, 16) est monté sur le filetage (7, 8) des broches (5, 6).

12. Dispositif de maintien de l'écartement selon les revendications 10 et 11, caractérisé en ce que le filetage (7, 8) de la broche (5, 6) présente au moins un méplat (9, 10), et en ce qu'une rondelle (11, 12) guidée sur le méplat en étant bloquée en rotation, est disposée entre le contre-écrou (15, 16) et le manchon (1), et comporte une denture frontale (13, 14) coopérant avec une face frontale du manchon (1).

13. Dispositif de maintien de l'écartement selon l'une des revendications 1 à 12, caractérisé en ce que les surfaces d'appui (26) présentent un contour rond.

14. Dispositif de maintien de l'écartement selon l'une des revendications 1 à 12, caractérisé en ce que les surfaces d'appui (26) présentent un contour de forme ovale.

15. Dispositif de maintien de l'écartement selon l'une des revendications 1 à 14, caractérisé en ce que les corps d'appui (23, 23′) comportent des tenons (28, 28′) faisant saillie sur les surfaces d'appui.

16. Dispositif de maintien de l'écartement selon la revendication 15, caractérisé en ce que chaque corps d'appui (23, 23′) comporte plusieurs tenons à proximité du bord et/ou du centre de la surface d'appui (26).

17. Dispositif de maintien de l'écartement selon l'une des revendications 15 ou 16, caractérisé en ce que chaque tenon (28, 28′) est arrondi à son extrémité libre.
